# EUROPEAN PATENT APPLICATION

(11) **EP 2 722 337 A1**
(43) Date of publication of application: **23.04.2014**
(21) Application number: 12189346.5
(22) Date of filing: 19.10.2012
(51) Int. Cl.: C07K 14/005, C12N 7/00, C12N 7/04, C12N 15/86, C12N 7/02

(54) **Production of a HCMV based vaccine in human amniocyte cell lines**

(71) Applicant: CEVEC Pharmaceuticals GmbH, 51105 Köln (DE)
(72) Inventor: Schiedner, Gudrun, Dr., 50733 Köln (DE); Plachter, Bodo, Prof. Dr., 55286 Wörrstadt (DE)
(74) Representative: Baudisch, Heidi

(57) **Abstract**

The present invention relates to a method for the production of HCMV particles, the method including the steps of: (a) contacting and thereby infecting a permanent human amniocyte cell with HCMV, (b) incubating the amniocyte cell, (c) allowing expression of HCMV particles, and (d) isolating of the HCMV particles, wherein the permanent human amniocyte cell expresses the adenoviral gene products E1A and E1B. Furthermore, the present invention relates HCMV particles produced by the method of the present invention as well as to a HCMV based vaccine comprising the HCMV particles, the use of the HCMV particles for use in the preparation of a HCMV based vaccine and the HCMV particles for use in the preparation of a therapeutic or diagnostic agent for the prevention or treatment of a HCMV related disease.

## Description

The present invention relates to a method for the production of human Cytomegalovirus (HCMV) particles, the method including the steps of: (a) contacting and thereby infecting a permanent human amniocyte cell with HCMV, (b) incubating the amniocyte cell, (c) allowing expression of HCMV particles, and (d) isolating of the HCMV particles, wherein the permanent human amniocyte cell expresses the adenoviral gene products E1A and E1B. Furthermore, the present invention relates to HCMV particles produced by the method of the present invention as well as to a HCMV based vaccine comprising the HCMV particles, the use of the HCMV particles for use in the preparation of a HCMV based vaccine and the HCMV particles for use in the preparation of a therapeutic or diagnostic agent for the prevention or treatment of a HCMV related disease.

Vaccination represents one of the most important means in health care system for the prevention of diseases. The success of the use of vaccination components is in particular dependent on the sufficient amount of vaccination material, for example attenuated viruses that should derive from stable and easily manageable sources.

In view of safety aspects, inactivated vaccines are advantageous since the components of such inactivated vaccines are non-infectious. In this context, subviral particles have been shown to be useful for vaccination. Distinct forms of such subviral particles are so called "Dense Bodies" (DB) which represent defective viral particles which are released during infection. Such DBs allow the use in form of an inactivated vaccine which is suitable to provoke favourable immune responses (5, 6- in the list of references). DBs of HCMV are described in EP 1 159 405.

DBs are released from cells that are infected with HCMV (2). Their size varies from 130-350nm (8). The inner protein structure is mainly composed of tegument proteins, pp65 (pUL83) and pUL25 being the most abundant constituents (10). The outer layer is made up by a lipid bilayer, derived from cellular membranes, in which viral glycoproteins are inserted (10). Besides abundant proteins, a number of further, less abundant polypeptides are contained in these particles. By virtue of the viral surface glycoproteins, DBs appear to enter fibroblasts by membrane fusion, comparable to virions (9).

Infection of healthy individuals with HCMV remains asymptomatic in most instances. Severe and life-threatening manifestations, however, may occur in immunocompromised patients, such as transplant recipients or individuals living with AIDS. Under such conditions, HCMV can infect multiple organs including lung, liver, gut, and salivary glands. HCMV is also one component of the "TORCH" complex which includes Toxoplasma gondii, Rubella virus, HCMV and Herpes simplex virus. Accordingly, HCMV can lead to congenital abnormalities in newborns and toddlers, following primary infection or, less likely, reactivation of HCMV during pregnancy. Sensorineural hearing loss, vision impairment and various degrees of mental disabilities are the most momentous manifestations in this setting. HCMV is a member of the *Beta-herpesviridae* family. These viruses are characterized by their strict species-specificity and their slow replication in cell culture. The genome of HCMV consists of a double-stranded DNA genome of about 230,000 base pairs, encoding a set of approximately 165 genes. The infectious HCMV-virions of about 200 nm in diameter are composed of an icosahedral capsid, containing the genome and a tegument layer. The tegument proteins define the matrix between the capsid and the outer viral envelope that consists of a lipid bilayer derived from cellular membranes. Viral glycoproteins, inserted into this matrix, are engaged in adsorption and penetration of host cells.

Viral tegument proteins, in particular pp65, have been identified as major target antigens of the T lymphocyte response against HCMV (11). Moreover, neutralizing antibodies are considered to be major effectors to prevent infection. Such antibodies are directed against HCMV surface glycoproteins, namely glycoprotein B (gB, gpUL55) or glycoprotein H (gH, gpUL75). pp65, gB, and gH are integral constituents of DB.

HCMV particles, such as DBs, are currently produced according to the prior art using human fibroblast cell cultures. The use of fibroblasts is however very complex. The use of fibroblast cell culture for clinical grade vaccine production is limited by lack of ready access to GMP-compliant fibroblast cells. In order to be approved by regulatory agencies, production cells need to be free of any infectious proteins and agents and their entire history must be documented. Usually fibroblast cells are non-transformed primary cells isolated from tissue. Among fibroblast cells, MRC-5 cells are best characterized for clinical production of vaccines and are derived from normal lung tissue of a 14-week-old male fetus isolated in 1966. MRC-5 cells grow as adherent cells in serum-containing medium and are capable of attaining 42-46 population doublings before onset of decline in proliferation. For production of vaccines used in patents, cells from a well-characterized and certified MCB with specific population doubling levels or passage levels need to be used. However, the limited population doublings of MRC-5 cells restricts the use of these cells in manufacturing to only a very small number of passages. Even though MRC-5 cells are used already for the production of numerous vaccines, they are not ease to use in large-scale production using bioreactor technology since they only grow as adherent cells and require the use of microcarriers or multilayer methods in serum-containing medium. In addition, they cannot be engineered in order to express complementing proteins for production of vaccine vectors that require complementation.

Furthermore, the culturing of human fibroblasts necessarily requires the addition of serum of animal origin, such as fetal calf serum, FCS, to the cell cultures. This is a disadvantage since the serum of animal origin is discussed to be a potentially harmful source which may deliver pathogens. The detection of such pathogens is however difficult or impossible. Particular concerns relate to the possible outcome of bovine spongiform encephalopathy (BSE) which is caused by misfolded proteins called prions. Further, fetal calf serum is a complex mixture containing unknown compounds and these can vary among different batches. In addition, well-known and unidentified proteins from animal source may cause allergies or side effects in patients. For production of clinical grade material, Good Manufacturing Practice (GMP) as defined by both the European Medicines Agency and Food and Drug Administration needs to be employed and require traceability of raw materials and consistency in batch composition. Therefore, FCS should not be used under GMP conditions, which are, however, required for the production of a safe and reliable vaccine or pharmaceutical composition. Thus, there is a demand to provide vaccine production methods which do not necessarily involve the use of potentially harmful substances.

In summary, the substitution of primary cells by a continuous serum-free cell line has numerous advantages over primary fibroblast cell culture.

Thus, there is a demand to provide vaccine production methods which do not necessarily involve the use of potentially harmful substances.

Therefore, the objective technical problem underlying the present invention is the provision of a method for the production of HCMV particles as well as the provision of a HCMV based vaccine which does not involve the use of potentially harmful substances such as animal serum.

This technical problem is solved according to the subject-matter as defined in the claims.

The invention is illustrated according to the following figures:

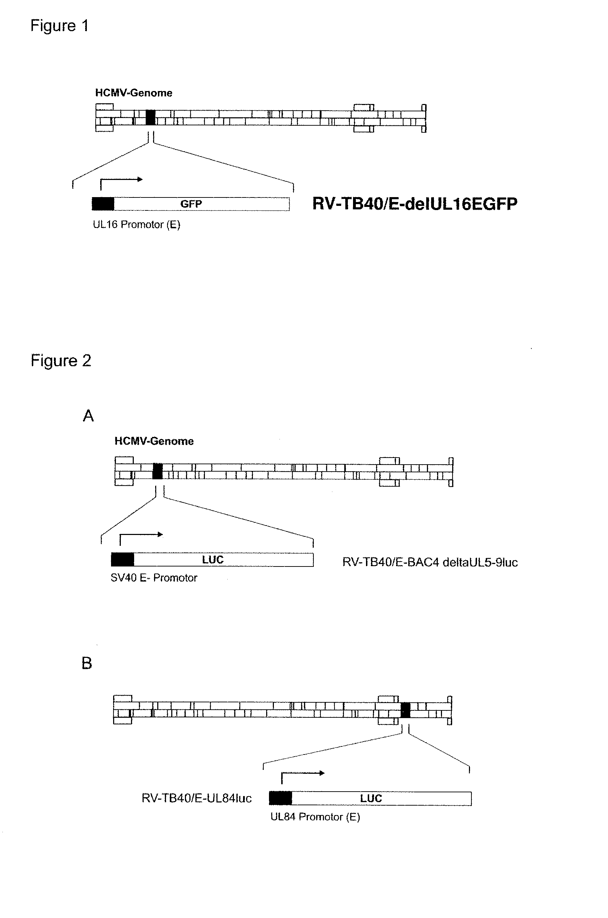

Figure 1 shows a schematic representation of the recombinant virus RV-TB40/E-delUL16EGFP. This virus is a derivative of HCMV strain TB40/E, expressing the Green fluorescent protein (GFP) under transcriptional control of the early UL16 - promotor of HCMV (3).
Figure 2 shows a schematic representation of recombinant viruses RV-TB40/E-BAC4 deltaUL5-9luc and RV-TB40/E-UL84luc. Fig. 2A, recombinant virus RV-TB40/E-BAC4 deltaUL5-9luc expresses the firefly luciferase under the control of the SV40 early promotor. The genomic region UL5-UL9 was replaced in constructing this virus (7). Fig. 2B, recombinant virus RV-TB40/E-UL84luc (generously provided by Thomas Stamminger, Erlangen) expresses the firefly luciferase under control of the early UL84 promotor of HCMV.
Figure 3 shows an indirect immunofluorescence (IF) analysis of CAP (Opti-Pro) cells and human foreskin fibroblasts (HFF), infected with the BAC - derived virus RV-TB40/E BAC7 (generously provided by Christian Sinzger, Ulm). Fig. 3A and Fig. 3C, indirect IF of infected CAP (Opti-Pro) cells, stained for IEl-pp72 expression (different magnifications). Fig. 3B and Fig. 3D, indirect IF of infected HFF, stained for IEl-pp72 expression (different magnifications).
Figure 4 shows a direct fluorescence analysis of CAP cells, infected with virus RV-TB40/E-delUL16EGFP. Fig. 4A, direct fluorescence of infected CAP (Opti-Pro) cells, kept in the presence of fetal calf serum. Fig. 4B, direct fluorescence of infected CAP cells (VP-SFM), kept in the absence of fetal calf serum.
Figure 5 shows Luciferase analyses of the infectability of adherent CAP (Opti-Pro) cells by HCMV at 24 hours of infection. Fig. 5A, representation of luciferase activity, measured following a 24 hour infection with RV-TB40/E deltaUL5-9luc. Fig. 5B, representation of luciferase activity measured following a 24 hour infection with RV-TB40/E-UL84luc.
Figure 6 shows Luciferase analyses of the infectability by HCMV of adherent CAP cells, kept in the presence of FCS (w FCS) or in the absence of FCS (w/o FCS). Fig. 6A, representation of luciferase activity, measured following a 48-hour infection with RV-TB40/E deltaUL5-9luc. Fig. 6B, representation of luciferase activity, measured following a 48-hour infection with RV-TB40/E-UL84luc.
Figure 7 shows the results of a quantitative PCR analysis of viral DNA contained in cells after infection of CAP (Opti-Pro) cells, CAP (VP-SFM) cells und HFF. The total number of viral genome copies in the dish in relation to time of infection is shown.
Figure 8 shows an Odyssey^{®} immunoblot analysis of the expression of viral proteins in CAP cells. Shown are two different images of the same blot using different exposure periods. The antibodies used were directed against pp65 or against a second tegument protein pp71.
Figure 9 shows the release of viral genomes into the cell culture supernatant of infected CAP cells or infected HFF, measured by quantitative PCR analysis. Infection was performed with RV-TB40/E-delUL16EGFP.
Figure 10 shows the release of viral genomes into the cell culture supernatant of infected CAP cells or MRC5, measured by quantitative PCR analysis (with medium exchange at day 1 of infection). Infection was performed with RV-TB40/E-delUL16EGFP.
Figure 11 shows an example of a direct GFP fluorescence of HFF, incubated with cell culture supernatants from 4-day infected CAP cells (left) or 7-day infected CAP cells (right). Microscopic inspection demonstrates the release of infectious virus from CAP cells.
Figure 12 shows the result of a plaque assay as measure for the release of infectious HCMV into the culture supernatant of infected CAP cells.

The terms "human Cytomegalovirus" or "HCMV" have to be understood according to the present invention as a species of virus which belongs to the viral family of *Herpesviridae.* HCMV, and CMVs from other mammals than humans, are cathegorized in the subfamily of *Beta-herpesviridae.* Alternative expressions for HCMV is also human herpesvirus-5 (HHV-5).

The expression "vaccine" according to the present invention relates a biologically or genetically generated antigen or a plurality of antigens, including proteins, protein subunits, peptides, carbohydrates, lipids, nucleic acids, inactivated or attenuated viruses, wherein the virus can be a complete virus particle or a part of a virus particle or combinations thereof. The antigen represents at least one epitope, for example a T cell and/or B cell eptiope. The antigen is recognized by immunological receptors, such as T cell receptors or B cell receptors. Accordingly, the vaccine activates, after its recognition, the immune system to target, for example, a distinct virus. An immunological response is provoked by this e.g. against viruses or viral antigens, this results in the development of antibodies and specialized T helper cells and cytolytic T cells which provide a long lasting protection. These effectors may endure between a few years and life-long, depending on the virus and depending on the respective antigen or vaccine. In the case of HCMV, viral reactivation may be suitable to boost the vaccine-induced immune response later in life. Vaccines include life and inactivated vaccines. Life vaccine comprises for example attenuated, but replication competent viruses, which are apathogenic. In the case of inactivated vaccines, viruses are killed or the vaccine only includes parts of the virus, such as antigens. The inactivation - killing - of viruses can be conducted with chemical substances, for example formaldehyde, beta-propiolactone and psoralene. The viral envelope is preserved in the course of that treatment. Furthermore, toxoid vaccines are existing which only contain the biologically inactive component - toxoid - of the toxin of an agent, for example the tetanus toxoid. The toxoid is also considered as an inactivated vaccine. Inactivated vaccines may also be composed of subfragments of viral envelope proteins. The destruction or cleavage of the virus envelope can be conducted by using detergents or strong organic solvents. Finally, inactivated vaccine further include subunit vaccines which are composed of specific components of a virus.

The expression "HCMV based vaccine" according to the present invention relates to all proteins, peptides or parts thereof as well as nucleic acids coding for said proteins, peptides or parts thereof of HCMV, and the HCMV particles itself, recombinant HCMV particles, including HCMV envelope proteins, subviral particles, virus-like particles (VLP), VLP complexes and/or parts thereof which can be used for immunization purposes against a HCMV infection.

The expression "adjuvant" according to the present invention relates to substances which are able to modulate the immunogenicity of an antigen. Adjuvants are in particular mineral salts, squalen mixtures, muramyl peptides, saponin derivates, preparations of the cell wall of mycobacteria, distinct emulsions, monophosphoryl-lipid-A, mycolic acid derivatives, non-ionic block-copolymer tensides, quil A, subunit of the choleratoxin B, polyphophazene and its derivatives, immunostimulatory complexes, cytokine adjuvants, MF59 adjuvants, mucosal adjuvants, distinct bacteria exotoxines, distinct oligonucleotides and PLG.

The expression "amniocyte" according to the present invention relates to all cells, which are present in the amniotic fluid of human origin and can be harvested via amniocentesis. These cells are derived from the amnion or from fetal tissue, which is in contact with the amniotic fluid. Three main classes of amniocyte cells are described which can be differentiated on the basis of morphological characteristics: fibroblast-like cells (F-cells), epitheloide cells (E-cells) and amniotic fluid cells (AF-cells)(4). AF-cells represent the dominant cell type.

The expression "permanent cells" or "permanent cell lines" according to the present invention relates to cells which are genetically altered such that a continuous growth in cell culture is possible under suitable culture conditions. These cells are also called immortalized cells.

The expression "primary cells" according to the present invention relates to cells which have been provided via direct removal from an organism or a tissue, and the cells are subsequently cultured. Primary cells posses only a limited life time.

The expression "adherent cell" according to the present invention relates to cells which only replicate when attached to surfaces either of microcarriers or to cell culture dishes. Usually, the attachement and the replication of adherent cells occurrs in the presence of serum. Adherent cells first need to be detached from the surface in order to be used to seed new cultures.

The expression "suspension cell" according to the present invention relates to cells which can be cultivated in suspension without beeing attached to surfaces. Usually, the cultivation of the suspension cells can be conducted in the absence of serum. Suspension cultures can easily be passaged without the need of detaching agents.

The expression "transfection" according to the present invention relates to any method which is suitable to deliver a distinct nucleic acid or nucleic acids in cells. For example transfection can be conducted using calcium phosphate, electroporation, liposomal systems or any kind of combinations of such procedures.

The expression "CAP" or "CAP cells" according to the present invention relates to permanent human amniocyte cell lines which have been generated via immortalization of primary human amniocytes with adenoviral gene functions E1A and E1B.

The expression "CAP-T" cells according to the present invention relates to CAP cells which have been additionally stably transfected with a nucleic acid molecule including the sequence of SV40 large T antigen.

The first subject-matter of the present invention relates to a method for the production of HCMV particles, the method including the steps of: (a) contacting and thereby infecting a permanent human amniocyte cell with HCMV, (b) incubating the amniocyte cell, (c) allowing expression of HCMV particles, and (d) isolating the HCMV particles, wherein the permanent human amniocyte cell expresses the adenoviral gene products E1A and E1B.

After infection of the cell with HCMV, HCMV replicates inside the cell and viral proteins are expressed. The expressed proteins are then assembled into viral particles. The assembled HCMV particles may be localized intracellularily in the intracellular space of the amniocyte cell or may be released from the cell into the medium. According to the present invention, step (c) allowing the expression of HCMV particles involves the replication of HCMV DNA, HCMV protein expression and assembly of HCMV particles.

In a preferred embodiment of the present invention, it is foreseen that the amniocyte cells in step (a) are cultured in serum free medium.

In a further preferred embodiment of the present invention, it is foreseen that the amniocyte cells in step (a) are cultured in serum-containing medium.

In a preferred embodiment of the present invention, it is foreseen that the amniocyte cells are adherent cells or suspension cells.

The culturing of the amniocyte cells in a medium without the necessity to add serum, in particular serum deriving from animal origin such as fetal calf serum - FCS, is advantageous. Serum with animal origin is potentially harmful since pathogens may reside in the serum which can then lead to diseases. Since such pathogens are difficult to be detected, it is desirable to avoid serum as addition in the culture medium to prevent any contamination of the products produced in cell culture. Moreover, serum such as FCS includes an undetermined number of unidentified proteins. Such proteins may provoke allergies or side-effects.

In a further preferred embodiment of the present invention, it is foreseen that the HCMV particles in step (c) are isolated from the medium or from the intracellular space of the amniocyte cell.

The isolation and purification of the HCMV particles produced according to the method of the present invention is conducted via standard procedures which are known in the prior art. The kind of purification is dependent on the origin of the HCMV particles. In the case the particles have to be recovered from the inside of the cells, since the HCMV particles are still intracellularly localized, it is necessary to permeabilize the cells. This permeabilization can be conducted due to shear forces or via osmolysis. Afterwards, insoluble material such as cell membranes is separated for example via centrifugation. Centrifugation is generally used to separate cells, cell organelles and proteins. Furthermore, after the separation of other cell components it is necessary to separate distinct proteins, peptides and amino acids of different size. The purification is negatively influenced by the presence of lipids and the presence of proteases. Such deactivation of proteases is important for the purification procedure. Proteins deriving from the extracellular matrix do not have to be extracted for the purification. However, after the separation of all insoluble components, the proteins derived from the extracellular matrix are very diluted and thus only present in minor amounts compared to intracellular deriving proteins.

Preferably, it is foreseen that the isolating in step (d) is conducted from the medium with rate velocity gradient centrifugation, density gradient differential centrifugation or zone centrifugation. Further alternative isolation methods are preferred which are suitable for the isolation of biomolecules like viruses. In particular preferred are alternative isolating steps which include the use of chromatography media that are cast as single units and result in fractionating large biomolecules like viruses.

In a preferred embodiment of the present invention, it is foreseen that the permanent human amniocyte cell is grown in the lag phase, the exponential phase or the stationary phase during the time of the contacting and infecting with HCMV in step (a).

Advantageously, infection efficiency is improved if the cells are infected during the exponential - also called log - phase and the stationary phase of growth.

In a further preferred embodiment of the present invention, cells are cultivated in a growth medium optimized for infection with HCMV. Alternatively, cells are cultivated in a medium optimized for high-cell denisity growth of cells, and for infection with HCMV, medium is exchanged completely or is diluted with a medium optimized for infection with HCMV.

Medium optimized for infection of cells with HCMV advantageously does not contain factors preventing or reducing infection of cells with HCMV by inhibiting virus-cell binding and/or fusion. These inhibitory factors include, but are not limited to, sulfated polysaccharides, antifoaming agents, agents avoiding shear stress of cells and hydrolysates.

For infection, cell concentrations in logarithmic growth phase are at least between 3x10⁵ cells/ml and up to 1x10⁷ cells/ml. In order to prolong the log-phase beyond 1x10⁷ cells/ml additional feed supplements or process operations can be applied prior to infection to achieve a high-cell-density concentrations. These supplements or process operations include glucose, glutamine, amino acids, or avoiding metabolic waste that limit cell growth, optimizing pH and osmolarity.

The permanent human cells used in the method according to the present invention are developed via immortalization of primary human cells. Primary human cells are yielded via direct removal from the organism or a tissue derived from the organism; removed cells are cultured. Particularly preferred are primary human cells which are altered to permanent human cell lines due to the expression of cell transforming factors. Preferred primary cells are amniocytes, embryonal retinal cells as well as embryonal cells of neuronal origin.

Cell transforming factors can be T-Antigen of SV40 (Genbank Acc. No. J02400), E6 and D7 gene products of HPV (e. g. HPV16, Genbank Acc No. K02718) and E1A and E1B gene products of human adenovirus (e.g. human Adenovirus Serotyp-5, Genbank Acc. No. X02996). The primary cells become immortalized due to the expression of E1 proteins of the human adenovirus through the transfection of both nucleic acid sequences for the E1A and E1B gene products. During the expression of the naturally occurring HPV, E6 and E7 can be expressed from one RNA transcript. The same applies for the expression of E1A and E1B of a naturally occurring adenovirus. The cell transforming factors, such as the adenoviral E1 gene function exert the immortalization or transformation and thus provide the enduring ability to culture the cells.

Immortalisation of primary cells occurs by transfecting cells with nucleic acid sequences expressing the respective transforming factors. Such nucleic acid sequences can be combined on one plasmid or located on several plasmids each containing expression units for single proteins. Expression units for transforming factors each comprises a promoter, a nucleotide sequence coding for the transforming factor and a 3' UTR. Nucleic acid molecules for transfection can also include fragments of the respective viral genome, e. g. the adenoviral genome, with the respective gene functions, e. g. E1A, E1B. The expression of the cell transforming factors can be conducted under the control of a homologous promoter or a heterologous promoter. As heterologous promotors can serve e. g. CMV (Cytomegalovirus) promotor, (Makrides, 9-26 in Makrides (ed.), Gene Transfer and Expression in Mammalian Cells, Elsevier, Amsterdam, 2003), EF-1α-promotor (Kim et al., Gene 91:217-223, 1990), CAG-Promotor (a hybrid promotor from the Immediate Early-Enhancer of human Cytomegalovirus and of a modified chicken β-action promotor with a first intron) (Niwa et al., Gene 108:193-199, 1991), human or murine pgk- (phosphoglyceratkinase-)promotor (Adra et al., Gene 60:65-74, 1987), RSV- (Rous Sarkoma Virus-) promotor (Makrides, 9-26 in: Makrides (ed.), Gene Transfer and Expression in Mammalian Cells, Elsevier, Amsterdam, 2003) or SV40- (Simian Virus 40-) promotor (Makrides, 9-26 in: Makrides (ed.), Gene Transfer and Expression in Mammalian Cells, Elsevier, Amsterdam, 2003).

The cells become immortalized due to the transfection of the primary human cells with a nucleic acid molecule including the E1A and E1B coding nucleic acid sequences. The nucleic acid molecule including E1A and E1B nucleic acid sequences used for the immortalization of the primary cells, are preferably from human adenovirus, in particular preferred from human adenovirus serotyp-5. In a particular preferred embodiment of the present invention, the nucleic acid molecule comprises besides of the E1A and E1B coding nucleic acid sequences further nucleic acid sequences coding for the adenoviral pIX gene function. The pIX polypeptide, a viral structural protein, functions as transcription activator for several virals and cellular promotors, such as thymidine kinase and beta-globin promotor. The transcription activating function of the pIX polypeptide additionally expressed in the cell may exert an elevation of the expression rates of the recombinant polypeptide, in the case the coding sequences of the recombinant polypeptide are under the control of one of the previously mentioned promotors in the cell lines according to the present invention. An example for such a sequence is disclosed in Genbank Acc No. X02996.

In a preferred embodiment of the present invention it is foreseen that the adenoviral gene products E1A und E1B comprise the nucleotide 1 to 4344, 505 to 3522 or nucleotide 505 to 4079 of the human adenovirus serotype-5.

In a preferred embodiment, the nucleic acid molecule for the immortalization of the primary cells comprises, in particular for amniocytes as primary cells, the adenovirus sertoype 5 nucleotide sequence of nucleotide 505 to nucleotide 4079. In a further particularly preferred embodiment of the present invention, the nucleic acid molecule used for immortalization of the primary cells, in particular of amniocytes, comprises the adenovirus serotype 5 nucleotide sequence of nucleotide 505 to nucleotide 3522. In a further particularly preferred embodiment the nucleic acid molecule used for the immortalization of primary cells, in particular of amniocytes, comprises the adenovirus serotype 5 nucleotide sequence of nucleotide 1 to nucleotide 4344, which corresponds to the adenoviral DNA in HEK-293 (Louis et al., Virology 233:423-429, 1997). Further, the immortalized human cell is able to express a viral factor which can bind to the origin of replication (ori) of a nucleic acid molecule which has been transfected into the cell. Due to this binding the replication of episomal nucleic acid molecules can be initiated. The episomal replication of nucleic acid molecules, in particular of plasmid DNA, in the cells exerts a strong augmentation of the number of copies of the transferred nucleic acid molecules and thus an elevation of the expression of a recombinant polypeptide encoded on the molecule as well as its maintenance over several cell divisions. Such a replication factor is for example the T-Antigen of Simian Virus 40 (SV40), which initiates the replication of the nucleic acid molecule, e. g. the plasmid DNA, after binding of a sequence which is designated as SV40 origin of replication (SV40 ori). The Epstein-Barr-virus protein EBNA-1 (Epstein Barr virus Nuclear Antigen-1) recognizes a so called ori-P origin of replication and catalyses the extrachromosomal replication of the ori-P including nucleic acid molecule. The T-Antigen of Simian Virus (SV40) activates not only as a replication factor the replication, but has also an activating effect on the transcription of some viral and cellular gene (Brady, John and Khoury, George, 1985, Molecular and Cellular Biology, Vol. 5, No. 6, p. 1391 to 1399).

The immortalized human cell used in the method according to the present invention is in particular an immortalized human amniocyte cell. In a preferred embodiment of the present invention, the immortalized human cell used in the method according to the present invention expresses the large T-Antigen of SV40 or the Epstein-Barr-virus (EBV) Nuclear Antigen-1 (EBNA-1). In a further preferred embodiment, the immortalized human cell used in the method according to the present invention, in particular the amniocyte cell, expresses the large T-Antigen of SV40 under the control of CAG, RSV or CMV promotor.

In a particularly preferred embodiment of the present invention, it is foreseen that the human permanent amniocyte cells are CAP cells. In a further particularly preferred embodiment of the present invention, it is foreseen that the human permanent amniocyte cells are CAP-T cells. These permanent human amniocyte cell lines are in particular disclosed in EP 1 230 354 and EP 1 948 789. In a further preferred embodiment, the human permanent amniocyte cells are N52.E6 cells. The N52.E6 cells have been deposited on 26 October 1999 at the Deutsche Sammlung von Mikroorganismen und Zellkulturen GmbH (DSMZ) according to the Budapest Treaty under the accession number DSM ACC2416.

Preferably, the permanent human amniocyte cell expresses the adenoviral gene product pIX. In a particularly preferred embodiment, the permanent human amniocyte cells are CAP cells which have been transfected with a murine pgk promotor, Ad5 sequences nt. 505-3522 comprising the whole E1 region, the 3' splice and polyadenylation signal of SV40 and the pIX region of Ad5 nt. 3485-4079. This plasmid is described in detail in EP 1 948 789.

In a preferred embodiment of the present invention, it is foreseen that the amniocyte cell is contacted and infected in step (a) with HCMV in an amount in the range of 0.001 to 10 MOI.

A further subject-matter of the present invention relates to the HCMV particles produced according to a method of the present invention.

Dense Bodies (DBs) are HCMV particles which are composed of pp65, gB, and gH as integral constituents. The HCMV particles represent defective viral particles which are released during infection. The HCMV particles possess an inner protein structure which is mainly composed of tegument proteins pp65 (pUL83) and pUL25. These proteins represent the most abundant proteins. The outer layer of these HCMV particles are composed of a lipid bilayer, derived from cellular membranes, in which viral glycoproteins are inserted. Further, less abundant proteins are present within the HCMV particles. Of particular importance is the protein pp65, which represent the major target protein to provoke a T lymphocyte response. Further, glycoprotein B (gB, gpUL55) and glycoprotein H (gH, gpUL75) are crucial since these glycoproteins are target structures of neutralizing antibodies.

A further subject-matter of the present invention relates to a HCMV based vaccine comprising HCMV particles according to the present invention.

A further subject-matter of the present invention relates to the use of HCMV particles according to the present invention for the preparation of a HCMV based vaccine.

The HCMV particles produced according to the method of the present invention, include HCMV proteins which are correctly folded. Thus, the protein folding is such that the appearance is comparable to the folding which occurs in a typical HCMV infection. This has the advantage that the HCMV particles included in the HCMV based vaccine are internalized by the cells of the subject as the recipient of the vaccine. After the internalization of the HCMV particles the viral proteins included in the particles are presented by antigen presenting cells (APCs). Due to the correct folding of the proteins, presentation by the APCs is conducted very efficiently. This in turn enables a strong immune response after the vaccination. The native folding of the proteins also allows the efficient induction of conformation-dependent, antiviral neutralizing antibodies, which may comprise a significant fraction of the total neutralizing-antibody capacity induced following natural HCMV infection.

Preferably, it is foreseen that the HCMV particles are placed in a pharmaceutically acceptable solution for the preparation of a HCMV based vaccine.

The HCMV based vaccine according to the present invention can be provided with one or more additional substances, such as stabilizers, neutralizers, carrier or substances for preservation. Such substances are for example formaldehyde, thiomersal, aluminium phosphate, acetone and phenol. Furthermore, the HCMV based vaccine according to the present invention may also include adjuvants to improve the immune stimulatory effect of the vaccine. Preferably, such adjuvants do not exert itself a pharmacological effect. These adjuvants may serve as solubilizer, emulsion or mixtures thereof. Adjuvants are for example mineral salts, squalen mixtures, muramyl peptides, saponin derivatives, preparations of Mycobacteria cell wall, distinct emulsions, monophosphoryl-lipid-A, mycolic acid derivatives, non-ionic block-copolymer tensides, quil A, subunit of cholera toxin B, polyphosphazene and its derivatives, immune stimulatory complexes, cytokine adjuvants, MF59 adjuvants, lipid adjuvants, mucosal adjuvants, distinct bacterial exotoxines, and distnct oligonucleotides and PLG.

A further subject-matter of the present invention relates to the HCMV particles according to the present invention for use in the preparation of a therapeutic or diagnostic agent for the prevention or treatment of HCMV related disease.

### List of references

1. Andreoni, M., M. Faircloth, L. Vugler, and W. J. Britt. 1989. A rapid microneutralization assay for the measurement of neutralizing antibody reactive with human cytomegalovirus. J.Virol.Methods 23:157-167.
2. Craighead, J. E., R. E. Kanich, and J. D. Almeida. 1972. Nonviral microbodies with viral antigenicity produced in cytomegalovirus-infected cells. J.Virol. 10:766-775.
3. Digel, M., K. L. Sampaio, G. Jahn, and C. Sinzger. 2006. Evidence for direct transfer of cytoplasmic material from infected to uninfected cells during cell-associated spread of human cytomegalovirus. J.Clin.Virol. 37:10-20. doi:S1386-6532(06)00160-0 [pii];10.1016/j.jcv.2006.05.007 [doi].
4. Hoehn, H., E. M. Bryant, L. E. Karp, and G. M. Martin. 1974. Cultivated cells from diagnostic amniocentesis in second trimester pregnancies. I. Clonal morphology and growth potential. Pediatr.Res. 8:746-754. doi:10.1203/00006450-197408000-00003 [doi].
5. Pepperl, S., J. Münster, M. Mach, J. R. Harris, and B. Plachter. 2000. Dense bodies of human cytomegalovirus induce both humoral and cellular immune responses in the absence of viral gene expression. J.Virol. 74:6132-6146.
6. Pepperl-Klindworth, S. and B. Plachter. 2006. Current perspectives in vaccine development, In: M. J. Reddehase (ed.), Cytomegaloviruses: Molecular Biology and Immunology. Caister Academic Press Ltd, Wymondham, Norfolk, U.K..
7. Scrivano, L., C. Sinzger, H. Nitschko, U. H. Koszinowski, and B. Adler. 2011. HCMV spread and cell tropism are determined by distinct virus populations. PLoS.Pathog. 7:e1001256. doi:10.1371/journal.ppat.1001256 [doi].
8. Stinski, M. F. 1976. Human cytomegalovirus: glycoproteins associated with virions and dense bodies. J.Virol. 19:594-609.
9. Topilko, A. and S. Michelson. 1994. Hyperimmediate entry of human cytomegalovirus virions and dense bodies into human fibroblasts. Res.Virol. 145:75-82.
10. Varnum, S. M., D. N. Streblow, M. E. Monroe, P. Smith, K. J. Auberry, L. Pasa-Tolic, D. Wang, D. G. Camp, K. Rodland, S. Wiley, W. Britt, T. Shenk, R. D. Smith, and J. A. Nelson. 2004. Identification of proteins in human cytomegalovirus (HCMV) particles: the HCMV proteome. J.Virol. 78:10960-10966.
11. Wills, M. R., A. J. Carmichael, K. Mynard, X. Jin, M. P. Weekes, B. Plachter, and J. G. Sissons. 1996. The human cytotoxic T-lymphocyte (CTL) response to cytomegalovirus is dominated by structural protein pp65: frequency, specificity, and T-cell receptor usage of pp65- specific CTL. J.Virol. 70:7569-7579.

The following examples describe the present invention, however, these examples should not be considered as limiting.

### Example 1: Infection of CAP (Opti-Pro) cells and human foreskin fibroblasts (HFF) with BAC-derived virus RV-TB40/E BAC7

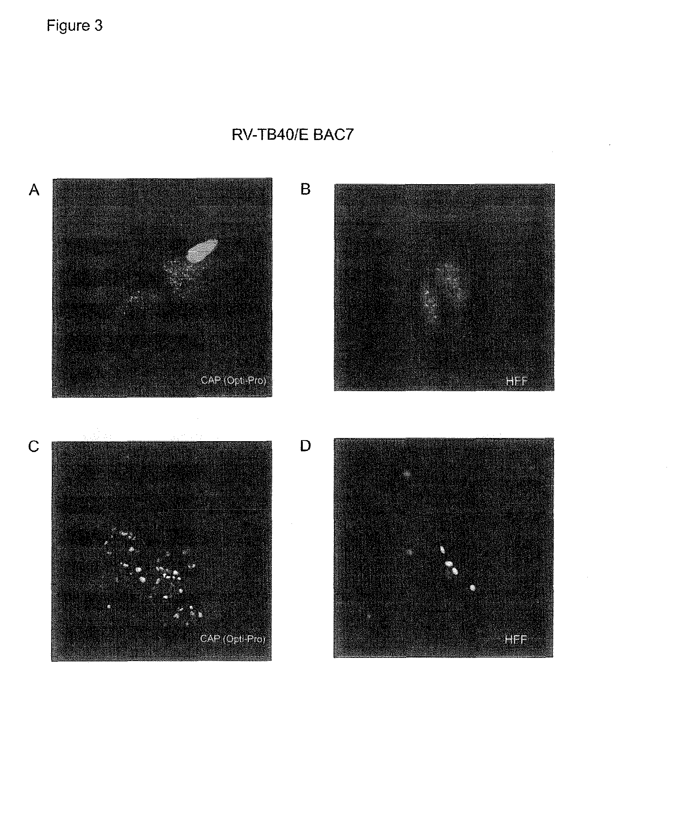

For this example 5 x 10⁵ cells were seeded in 10 cm culture dishes (78cm²). Coverslips were inserted into these dishes. Following overnight incubation, cells were infected with an m.o.i. of 1 with TB40/E BAC7 in a total volume of 4mL. After an adsorption period of 1.5 hours, cells were replenished with medium to a total volume of 10mL for each dish. A mock-infected control was carried along for each cell type. One day post infection, cover slips were collected, washed with 1XPBS and fixed in acetone p.a. (AppliChem; A1600,2500) for 1 hour at room temperature. Subsequently, the cell membrane was permeabilized by several rounds of rinsing in 1XPBS/0.1% Triton (3 times; TritonX 100; Roth). After that, cells were incubated with murine monoclonal antibody p63-27, directed against the HCMV protein IE1-pp72 [(ppUL123; (1)] for 1h, 37°C. Cells were then washed several times in 1XPBS/0.1% Triton (3 times) and incubated with a FITC-labeled secondary antibody, directed against mouse immunoglobulin for 1 hour, 37°C. For staining of the nuclei, DAPI (4',6-Diamidin-2-phenylindol; Invitrogen) was diluted 1:5,000 in 1XPBS and was added to the cover slips in a volume of 150µL (together with the secondary antibody, final volume of 300µL). Cells were then kept at RT in the dark for 10 minutes. After that, cover slips were rinsed three times in 1XPBS/0.1% TritonX 100 and once with H₂O_{dest.}. After that, coverslips were transferred to glass slides and fixed with Mowiol (Sigma Aldrich; 81381-250g; 20g dissolved in 80mL 1XPBS and 40mL glycerol). Glass slides were kept in the dark at room temperature to allow drying until inspection. Fig. 3A and Fig. 3C, indirect IF of infected CAP (Opti-Pro) cells stained for IE1-pp72 expression (different magnifications). Fig. 3B and Fig. 3D, indirect IF of infected HFF, stained for IE1-pp72 expression (different magnifications).

### Example 2: Infection of CAP cells with virus RV-TB40/E-delUL16EGFP

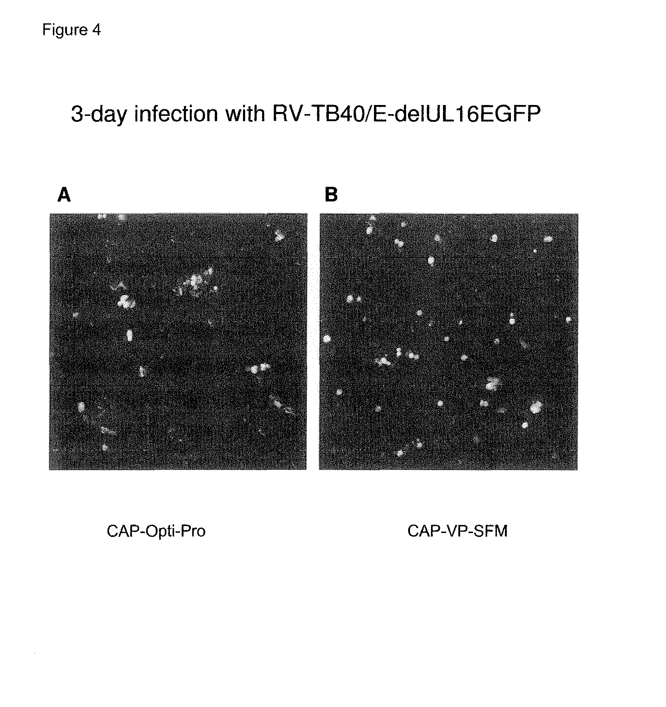

For this example 5 x 10⁵ cells were seeded in 75cm² culture bottles. Following overnight incubation, cells were infected with an m.o.i. of 1 with virus RV-TB40/E-delUL16EGFP in a total volume of 4mL. After an adsorption period of 1.5 hours, cells were replenished with medium to a total volume of 10mL for each flask. Three days post infection, infected cells were inspected *in vivo,* using a Leitz DMIRB microscope (luminous source: Leica 220V; 50/60HZ, HG 50W). Fig. 4A shows a direct fluorescence of infected CAP (Opti-Pro), kept in the presence of fetal calf serum. Fig. 4B shows a direct fluorescence of infected CAP (VP-SFM), kept in the absence of fetal calf serum.

The results shown in figures 3 and 4 provided the proof that HCMV can penetrate CAP cells and initiate its immediate - early gene expression. The latter is considered essential for lytic replication. About 20-40% of adherent CAP cells proved to be infectable (CAP Opti-Pro).

### Example 3: Luciferase analyses of the infectability of adherent CAP (Opti-Pro) cells by HCMV at 24 hours of infection.

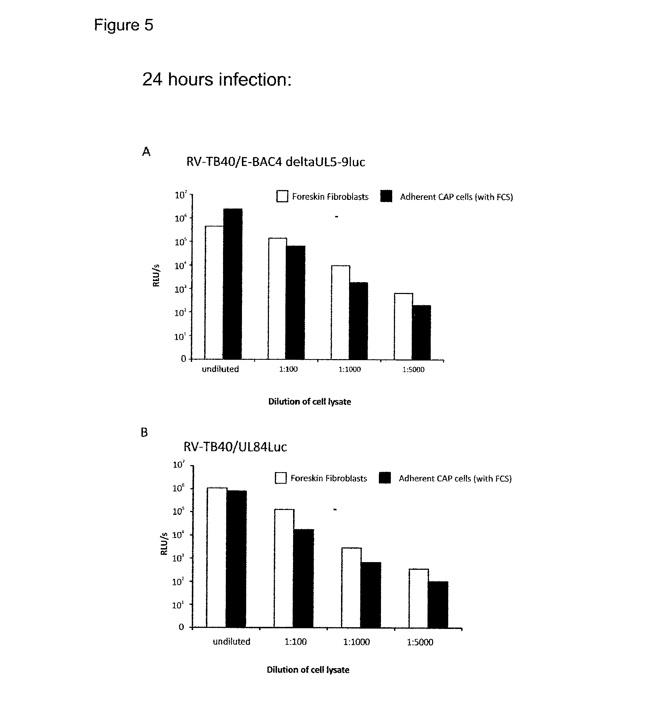

CAP (Opti-Pro) cells and HFF (control) were seeded in 96-well plates (1.5 x 10⁴ cells per well in 25µL). After overnight incubation, cells were infected with RV-TB40/E-BAC4 deltaUL5-9luc and RV-TB40/E-UL84luc, respectively at different dilutions (as indicated). After an incubation period of 24 hours, cells were cooled to room temperature. 125µL of BrightGlow^{™} reagent (Promega Bright Glow^{™} luciferase assay system) were added and cells were incubated for 2 minutes. Luciferase activity was measured in a Berthold Detection System Orin II Microplate Luminometer. Fig. 5A is a representation of luciferase activity, measured following a 24 hour infection with RV-TB40/E deltaUL5-91uc. Fig. 5B is a representation of luciferase activity measured following a 24 hour infection with RV-TB40/E-UL84luc.

### Example 4: Luciferase analyses of the infectability of adherent CAP (Opti-Pro) cells and CAP (VP-SFM) by HCMV at 48 hours of infection

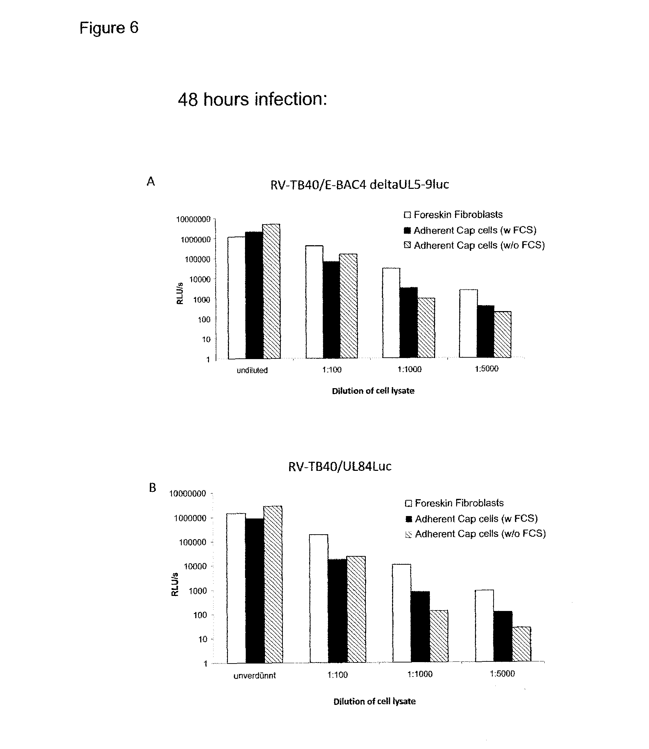

The experimental setup was as described in example 3. Fig. 6A is a representation of luciferase activity, measured following a 48 hour infection with RV-TB40/E deltaUL5-9luc. Fig. 6B is a representation of luciferase activity, measured following a 48 hour infection with RV-TB40/E-UL84luc.

The results shown in figures 5 and 6 confirmed the findings of figures 3 and 4 with respect to the infectability of CAP cells by HCMV. The result of figures 5B and 6B, in particular, showed that early promotors of HCMV (UL84) are active in CAP (Opti-Pro) and CAP (VP-SFM).

### Example 5: Determination of viral DNA after infection of CAP (Opti-Pro), CAP (VP-SFM) und HFF

Virus used for infection had to be normalized to genome copies in infected cells at 6 hours post infection. For this, 2.5 x 10⁶ CAP (Opti-Pro) cells and 2.5 x 10⁶ HFF were seeded in 10cm dishes (10 dishes total, 5 each per cell type). After overnight incubation, cells were infected in 3mL culture medium, using different virus dilutions (5µL, 10µL, 50µL, 100µL, 500µL) of a culture supernatant from TB40/E BAC7. After an adsorption period of 1.5 hours, dishes were replenished with additional 7mL of culture medium. At 6 hours after infection, supernatant was discarded. Cells were washed two times using 3mL of 1XPBS. Following that, 2.5mL of trypsin were added to HFF, spread over the dish and left for 5 minutes at 37°C. After that, trypsin was discarded. For CAP-cultures, 3mL of trypsin was applied, spread and discarded directly afterwards without incubation. After that, 2.5 to 3mL of medium containing FCS was added to stop the reaction. Following that, cells that were detached from the support, were centrifuged at 472xg for HFF and at 134xg for CAP cells for 5 minutes. The cell pellet was resuspended in 500µL 1XPBS. Cells were then counted and adjusted to 1 x 10⁶/mL. For this, cells were again centrifuged at 472xg (HFF) or at 134xg (CAP) and were then resuspended in the appropriate volume of 1XPBS. DNA was isolated using the "High Pure Viral Nucleic Acid Kit, Roche©" according to the manufacturer's instructions. Viral DNA concentration was determined in each sample using the ABI Prism 7700 Sequence Detection System (Serial-No.: 100000740).

Reagents:
Hot Star Taq polymerase (Quiagen; 5 U/µL)
Mastermix
10x PCR Buffer, including 15mM MgCl₂, 25mM MgCl₂, 2mM dNTPs, 0.3µM CMV-forward primer and reverse primer (Eurofins MWG Operon), 1µM probe (TIB MOLBIOL), 100µM ROX (6-Carboxy-X-rhodamin) in LiChrosolv water for chromatography (Merck, Cat.-No. 1.15333.1000).
1.2mL mastermix and 12.5µL polymerase were mixed.

Reactions were performed in 96 well plates in triplicates. The first vial received 1 35µL master mix. 15µL template was subsequently added. 50µL each of that mixture were transferred to the second and third vial. The tubes were sealed and applied to the ABI Prism 7700 Sequence Detection System. Program details were as follows:

| | |
|---|---|
| 50°C for 2 min | |
| 95°C for 5 min | (activation of Hot Star Taq) |
| 45 cycles of | |
| | |
| 94°C for 15 sec | (denaturation) |
| 60°C for 1 min | (annealing, elongation) |
| 4°C | End of reaction |

Results were calculated in genomes/mL. Using these results, volumes of virus stocks were determined which were necessary to provide 4 genome/copies per cell at 6 hours of infection.

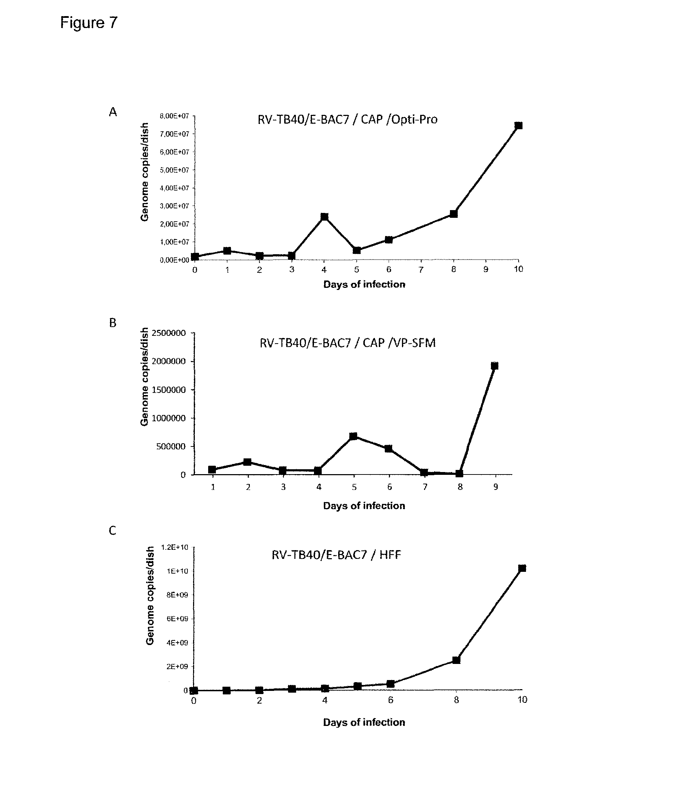

To determine replication kinetics in infected cells, 0.5 x 10⁶ HFF and CAP cells, respectively were seeded in 9x 10cm dishes. After incubation overnight at 37°C, cells were infected with TB40/E BAC7 to result in 4 genome copies of viral DNA per cell. After different intervals, cells were collected and counted. DNA was isolated and viral genome copies/cell were determined using the methodology as described above. Figure 7 shows the total number of viral genome copies in the dish in relation to time of infection.

The results - shown in fig. 7 - proved that HCMV can replicate its genome in CAP cells, irrespective to whether or not serum is added to the medium.

### Example 6: Odyssey^{®} immunoblot analysis of the expression of viral proteins in CAP cells

1.8 x 10⁶ CAP (Opti-Pro) cells were seeded 175cm² cell culture flasks. 1.5 x 10⁶ MRC5 were also seeded in parallel. After overnight incubation at 37°C/5%CO₂ in a humidified atmosphere, cells were infected with RV-40E-deltaUL16EGFP at a multiplicity of infection of 10 (CAP) or 1 (MRC5), respectively. At six days after infection, culture medium was removed and cells were washed once with 1XPBS. 5mL of trypsin was added and immediately removed (CAP) or left on the cells at 37°C for 5 minutes (MRC5). Addition of medium with FCS was used to stop the reaction. Cells were centrifuged at 472xg (MRC5) or 134xg (CAP OptiPro), 1xPBS was added (to wash the cells again). After that, cells where counted. After another centrifugation step, the cells were resuspended in Laemmli-Buffer (125mM Tris-Base, 2% vol/vol ß-mercaptoethanol, vol/vol 10% glycerin, 1mM EDTA pH 8.0, 0.005% vol/vol bromphenol-blue, H₂O_{dest}.) to result in 2 x 10⁵cells/10µL. Samples were boiled for 10 minutes, centrifuged at 1,300g for three minutes, partitioned and stored at -20°C until further use.

For the preparation of the sample "CAP (Opti-Pro 14dp.i. SN 40pL)", two 175 cm² flasks, containing an initial seed of 1.8 x 10⁶ CAP cells each were infected with RV-TB40/E-delUL16EGFP at an multiplicity of infection of 10 and were kept for 14 days at 37°C/5%CO₂ in a humidified atmosphere. A culture medium exchange was performed at one day after infection. After 14 days of infection, culture supernatant was collected and centrifuged at 1647xg for 10 minutes to remove cell debris. The supernatants were transferred to ultracentrifuge tubes and were centrifuged at 131.250xg, 10°C for 70 minutes in a Beckman Coulter Optima L-90K ultracentrifuge (Serial-No.: COL08L10). The supernatant was then removed carefully. The pellet was thoroughly resuspended in 40µL of Laemmli-Puffer. Again the sample was boiled for 10 minutes and was then centrifuged at 1,300 x g for 3 minutes. Samples were stored at -20°C until further use.

10% SDS-polyacrylamide gels were used for the separation of the proteins in the samples. Separation gels: 10ml H₂O_{dest}., 6,25ml Tris 1,5 M (pH 8.8), 8.3mL Gel 30 (Rotiphrese® Gel 30, Roth) 250µL 10% SDS, 250µL 10% APS, 15µL TEMED. Stacking gels: 4.30mL H₂O_{dest.}, 0.75mL Tris 1M (pH 6.8), 1.05mL Gel 30 (Acrylamide), 0.62µL 10% SDS, 0.62µ 10% APS, 7.5µLTEMED.

After polymerisation, gels were mounted on a vertical gel chamber (Hoefer SE 600 Series Electrophoresis Unit; US Patent 4,224,134). The running buffer (upper and lower buffer chamber) contained 1XPAGE buffer (200mL 5x PAGE-Puffer [25 mM Tris-Base, 192 mM glycine, 0.1% SDS] + 800mL H₂O_{dest.}). 10µL pre-stained protein marker (PeqGOLD Protein Marker IV from Peqlab; cat. no. 27-2110) was applied to the first slot; the samples were applied to the other slots. Proteins were separated overnight at 4,35V/cm (50 V, 400 mA und 100 W; Electrophoresis Power Supply - EPS 600; Pharmacia Biotech).

The following day, the glass plates were removed and the gels were rinsed in water. The PVDF filter (PVDF-Membrane: "Millipore" Immobilion, Transfer Membrane, Immobilion-FL, cat. no. IPFL00010) was cut in appropriate pieces and was rinsed in methanol for 5 minutes, briefly rinsed in water and then equilibrated for 20 minutes in transfer buffer/10% vol/vol methanol (25mM Tris, 192mM Glycin, 10% Methanol, 2L H₂O_{dest.}). The gel was also incubated in transfer buffer for 20 minutes. The initial layer on the semi-dry blot apparatus (Hölzel) consisted of three layers of chromatography paper (Chromatography Paper Whatman^{®}) which was pre-soaked in transfer buffer. Next, the equilibrated PVDF-membrane was applied, avoiding the generation of air-bubbles. After that the gel was applied, again avoiding air-bubbles. The final stack consisted of 3 layers of chromatography paper. The transfer was performed using 600V, 400mA for 75 minutes. After that, the apparatus was dismantled and the PVDF-membrane with the transferred proteins was air-dried for 1-2 hours in a fume hood. The membrane was then briefly rinsed in methanol, followed by rinsing it in H₂O. After an incubation in 1XPBS for a few minutes, blocking reagent (5% w/v dry milk powder [Roth, Art.Nr. T145.2] in 1XPBS) was applied and the filters were incubated on a tumbling device for 1 hour.

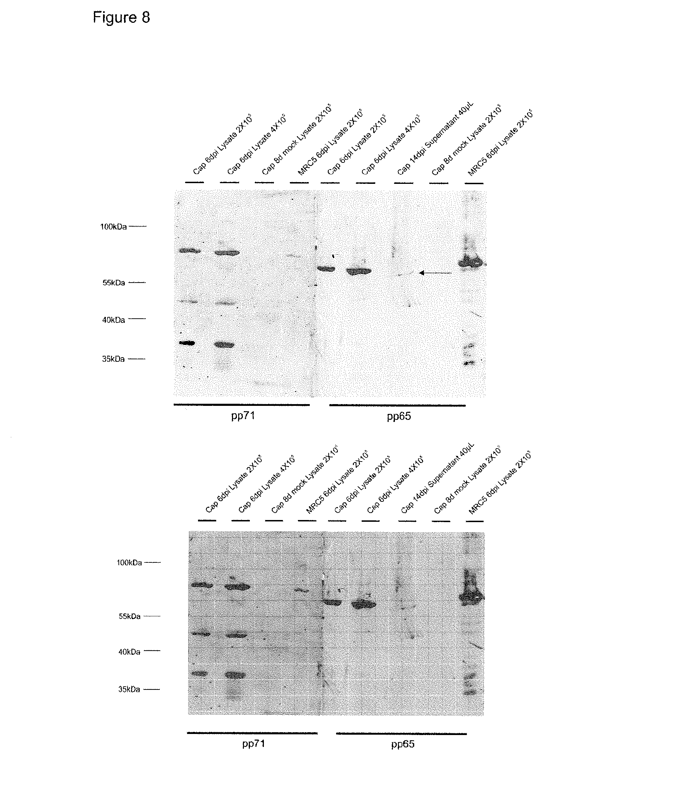

The primary murine monoclonal antibody 65-33, directed against pp65 (ppUL83) of HCMV (obtained from Prof. W. Britt, UAB, Birmingham, AL, USA) was diluted 1:500 in blocking reagent/0.1% v/v Tween100. Primary goat polyclonal antibodies against pp71 (ppUL82; vC-20, Santa Cruz Biotechnology, Heidelberg) were diluted 1:200 in blocking reagent/0.1% v/v Tween100. Membranes were incubated with the primary antibody in film wraps over night at room temperature, avoiding trapping for air bubbles. The next day, the antibody was removed and the filters were washed three times for 10 minutes in 1XPBS/0.2% v/v Tween100. After that, secondary antibodies were applied in a dilution of 1:5.000 in blocking reagent/0.1% v/v Tween100/0.01% v/v SDS. Secondary antibodies were IR800 donkey-anti-goat or IR800 goat-anti-mouse (Rockland). Incubation was performed in the dark for two hours at room temperature. After that, the secondary antibody was removed by 2 washing steps for 10 minutes in 1XPBS / 0.2% Tween100 and 1 washing step for 10 minutes in 1XPBS in the dark. Evaluation of the results was performed with an "Odyssey Imager"(LI-COR; Lincoln, NE, USA). In fig. 8, two 2 different images of the same blot with different exposure periods are shown.

The results of the immunoblots, illustrated in fig. 8, showed that pp65 and pp71 are detectable in infected CAP (Opti-Pro) cells at 6 days post infection and that pp71 was also faintly detectable in the supernatant of infected CAP cells, collected at 14 days post infection (arrow).

### Example 7: Release of viral genomes into the cell culture supernatant of infected CAP cells

2x 1.94 x 10⁶ human foreskin fibroblasts or 2x 1.8 x 10⁶ CAP (Opti-Pro) cells or 2x 1.6 x 10⁶ CAP (VP-SFM) were seeded in 175cm² culture flasks. 20mL of the appropriate culture medium was added. Incubation was over night at 37°C/5%CO₂ in a humidified atmosphere. Cells were infected with RV-TB40/E-delUL16EGFP the following day in a total volume of 5mL. For HFF and CAP (VP-SFM), an infectious dose of 0.1 (m.o.i.), for CAP (Opti-Pro) an infectious dose of 1 (m.o.i.) was used. After an adsorption period of 1.5 hours, 15mL of additional medium was added to the flasks. After that, 1.5mL culture supernatant was removed from each flask and stored at -20°C. The DNA concentration measured in these samples was taken as base-line value (see below). The volume that had been removed from the flasks was supplemented with fresh medium on this and all following occasions. At day 1 of infection, the culture medium in all flasks was replaced by fresh medium. At days 2, 3, 5, and 6 (or days 2, 3, and 6 for CAP VP-SFM), 1.5mL of culture supernatant was sampled from each flask and replaced by 1.5mL of fresh medium. At day 6, all cells were passaged and 2x 1.8 x 10⁶ of both HFF and CAP (Opti-Pro or VP-SFM) cells were seeded in new 175cm² flasks. Surplus cells were discarded. Again, 1.5mL of culture medium was sampled at days 8, 10, 12, 13, and 14 (or at days 8, 10, 13, and 14 for CAP VP-SFM).

The DNA contained in 200µL of each sampled specimens was extracted using the "High Pure Viral Nucleic Acid Kit, ROCHE^{©}" Kit according to the manufacturer's instructions. Quantification of viral genomic DNA was performed using ABI Prism 7700 Sequence Detection System, Applied Biosystems, (Serial-No.: 100000740).

Reagents:
Hot Star Taq polymerase (Quiagen; 5 U/µL)
Mastermix
10x PCR Buffer, including 15mM MgCl₂, 25mM MgCl₂, 2mM dNTPs, 3µM CMV-forward primer and reverse primer (Eurofins MWG Operon), 1µM probe (TIB MOLBIOL), 100µM ROX (6-Carboxy-X-rhodamin) in LiChrosolv water for chromatography (Merck, Cat.-No. 1.15333.1000).
1.2mL Mastermix and 12.5µL polymerase were mixed.

Reactions were performed in 96 well plates in triplicates. The first vial received 135 µL master mix. 15µl template was subsequently added. 50µL each of that mixture was transferred to the second and third vial. The tubes were sealed and applied to the ABI Prism 7700 Sequence Detection System. Program details were as follows:

| | |
|---|---|
| 50°C for 2 min | |
| 95°C for 5 min | (activation of Hot Star Taq) |
| 45 cycles of | |
| | |
| 94°C for 15 sec | (denaturation) |
| 60°C for 1 min | (annealing, elongation) |
| 4°C | End of reaction |

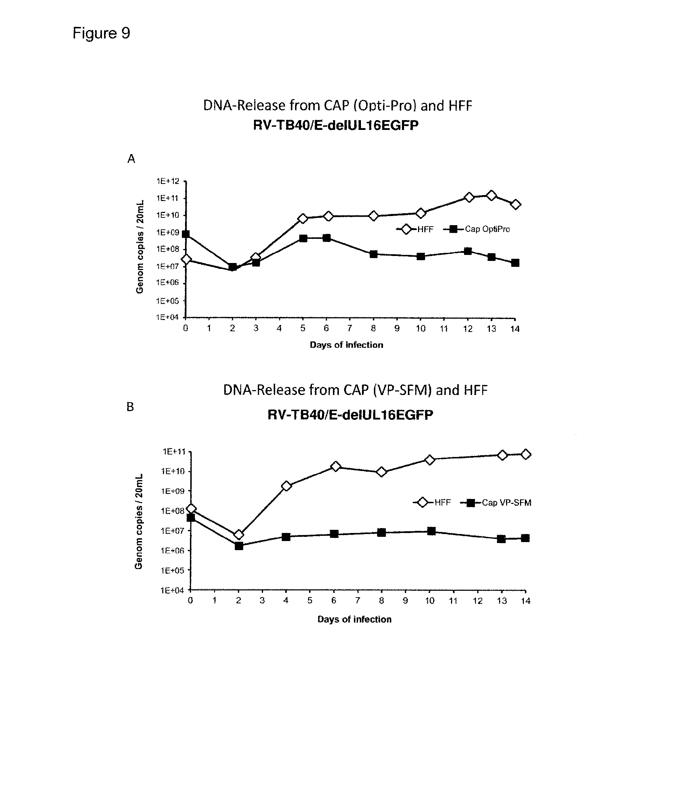

The results were calculated as genome copies/20mL of culture supernatant and are shown in fig. 9. This provided proof that viral genomes were released over a prolonged period from infected CAP (Opti-Pro) cells.

### Example 8: Release of viral genomes into the cell culture supernatant of infected CAP cells (with medium exchange at day 1 of infection)

1.5 x 10⁶ MRCS or 1.8 x 10⁶ CAP (Opti-Pro) cells were seeded in 175cm² culture flasks in quadruplicates. 20mL of the appropriate culture medium was added. Incubation was over night at 37°C/5%CO₂ in a humidified atmosphere. Cells were infected with RV-TB40/E-delUL16EGFP the following day in a total volume of 5mL. Infection was with an m.o.i. of 5 or 10, respectively for CAP (Opti-Pro), and 1 for MRC5 cells.

After an adsorption period of 1.5 hours, 15mL of additional medium was added to the flasks. After that, 1.5mL culture supernatant was removed from each flask and stored at -20°C. The DNA concentration measured in these samples was taken as basal value (see below). The volume that had been removed from the flasks was supplemented with fresh medium on this and all following occasions. At day 1 of infection, the culture medium in all flasks was removed, cells were rinsed in 1XPBS and medium was replaced by fresh medium. At days 4, 5, 6, 8, 11, 12, and 14, 1.5mL of culture supernatant was sampled from each flask and replaced by 1.53mL of fresh medium. Samples were stored at -20°C until further use.

The DNA contained in 200µL of each sampled specimens was extracted using the "High Pure Viral Nucleic Acid Kit, ROCHE^{©}"Kit according to the manufacturer's instructions. Quantification of viral genomic DNA was performed using ABI Prism 7700 Sequence Detection System, Applied Biosystems (Serial-No.: 100000740).

Reagents:
Hot Star Taq polymerase (Quiagen; 5 U/µL)
Mastermix
10x PCR Buffer, including 15mM MgCl₂, 25mM MgCl₂, 2mM dNTPs, 3µM CMV-forward primer and reverse primer (MWG), 1µM probe (TIB MOLBIOL), 100µM ROX (6-Carboxy-X-rhodamin) in LiChrosolv water for chromatography (Merck, Cat.-No. 1.15333.1000). 1.2mL Mastermix and 12.5µL polymerase were mixed.

Reactions were performed in 96 well plates in triplicates. The first vial received 135 µL Mastermix. 15µl template was subsequently added. 50µL each of that mixture was transferred to the second and third vial. The tubes were sealed and applied to the ABI Prism 7700 Sequence Detection System. Program details were as follows:

| | |
|---|---|
| 50°C for 2 min | |
| 95°C for 5 min | (activation of Hot Star Taq) |
| 45 cycles of | |
| | |
| 94°C for 15 sec | (denaturation) |
| 60°C for 1 min | (annealing, elongation) |
| 4°C | End of reaction |

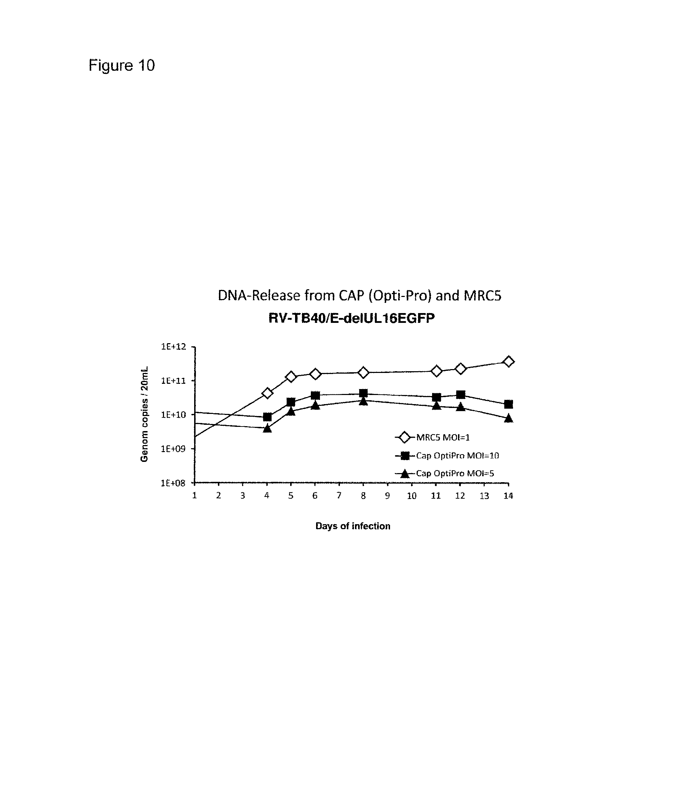

The results were calculated as genome copies/20mL of culture supernatant and are shown in fig. 10.

The results provided proof that viral genomes were released over a prolonged period from infected CAP (Opti-Pro) cells.

### Example 9: Proof of release of infectious virus from CAP cells

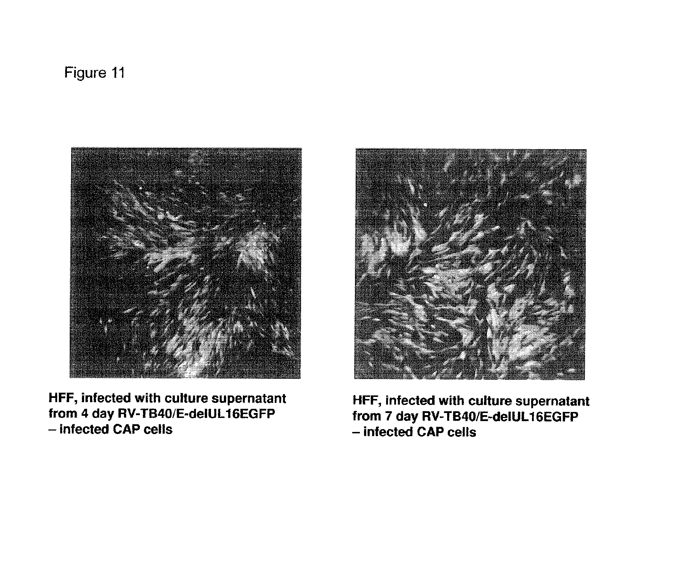

0.5 x 10⁶ human foreskin fibroblasts, or 1.2 x 10⁶ CAP (Opti-Pro) cells, or 1 x 10⁶ CAP (VP-SFM) cells, respectively, were seeded in 75cm² culture flasks and incubated at 37°C/5%CO₂ in a humidified atmosphere overnight. Cells were infected with RV-TB40/E-delUL16EGFP the following day at m.o.i.s of 0.5, 1, and 5. 1-1.5mL culture supernatant was collected at days 3, 6, and 7 and stored at -80°C until further analysis. 3 x 10⁵ HFF were seeded in 25cm² culture flasks and incubated overnight. The following day, HFF were infected with the supernatants. Figure 11 shows an example of the direct GFP fluorescence, detectable upon microscopic inspection.

This experiment proved that infectious virus was released from infected CAP cells.

### Example 10: Release of infectious HCMV into the culture supernatant of infected CAP cells, measured by plaque assay

2x 1.5 x 10⁶ MRCS cells or 4x 1.8 x 10⁶ CAP (Opti-Pro) cells were seeded in 175cm² culture flasks. 20mL of the appropriate culture medium was added. Incubation was over night at 37°C/5%CO₂ in a humidified atmosphere. Cells were infected with RV-TB40/E-delUL16EGFP the following day in a total volume of 5mL. For MRC5, an infectious dose of 1 (m.o.i.), for CAP (Opti-Pro) an infectious dose of 5 or 10 (m.o.i.) was used. After an adsorption period of 1.5 hours, 15mL of additional medium was added to the flasks. At day 1 of infection, the culture medium in all flasks was replaced by fresh medium. 1.5mL of medium was collected as initial sample and was replaced by 1.5mL of fresh medium. At days 6, 11, and 14, 1.5mL of culture supernatant was sampled from each flask and replaced by 1.5mL of fresh medium.

For measurement of infectious virus in the samples, a standard plaque assay was performed on human foreskin fibroblasts. For this, 3 x 10⁵ fibroblasts were seeded in 2mL of the culture medium in each well of 6-well plates (5 plates). Cells were cultured overnight as described above. 1mL of the samples was applied to the cells the following day. Supernatant from MRC-V was diluted by 1:10, supernatant from CAP cells was used undiluted or at a dilution of 1:10. Adsorption was for 1.5 hours at 37°C. The supernatant was then discarded. 4mL of overlay medium was applied to the cells to prevent viral spread. The plates were kept at room temperature for 30 minutes to allow solidification of the overlay. Following that, plates were incubated for 6 days at 37°C as described above. After that, the overlay was carefully removed and crystal violet solution was applied (crystal violet from Roth [25 g] 1% cristal violet / 50% Ethanol / H₂O_{dest.}).

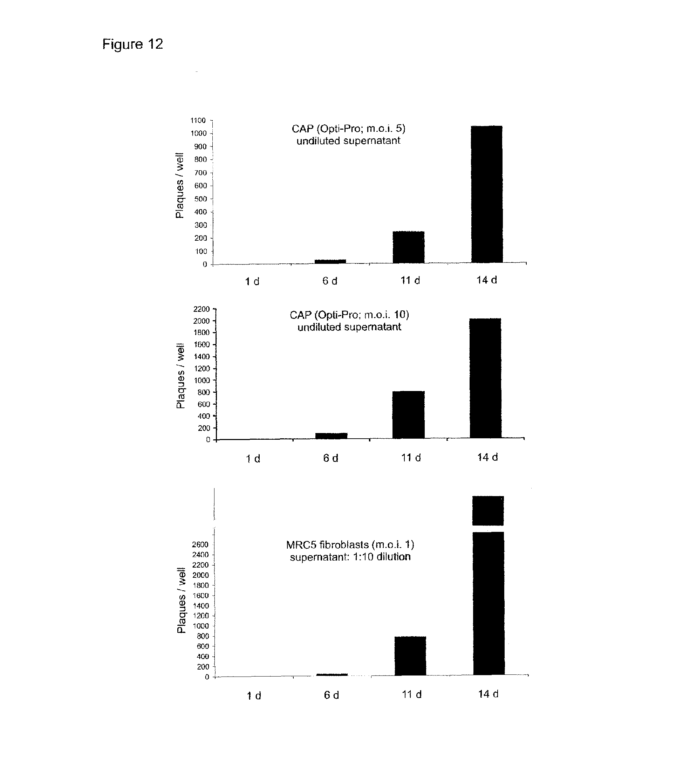

After an incubation of 10 minutes, the liquid was removed and wells were rinsed with tap water until the staining appeared appropriate. Plates were dried by leaving them on the shelf for one day. Plaques were then counted using a Leitz DMIRB microscope. The results of the plaque assay are shown in fig. 12.

### Preparation of overlay medium.

Prepare agarose stock:
Ad 1.5g agarose (Gibco) to 50mL of 1xPBS.
Heat this in a microwave oven.
Autoclave
Apportion 5mL each into 50mL Falcon tubes under sterile conditions.
Store at 4°C until use.

To prepare overlay with 0.3% w/v agarose, 5mL of stock agarose was briefly heated in a microwave oven and cooled to 42°C in a water bath. In parallel, culture medium (MEM) was also heated to 42°C. The solutions were allowed to cool at room temperature for 15 minutes (final temperature of 39-40°C). After adsorption, 5mL agarose and 45mL of the medium were mixed and were applied to the cells to prevent spread.

The results as depicted in fig. 12 showed that CAP (Opti-Pro) cells released infectious virus for a prolonged period of time.

### Example 11: Analysis of the induction of apoptosis by HCMV infection of CAP-cells

3 x 10⁴ CAP (Opti-Pro) cells were seeded in a *1u-Slide 8 well Coated (poly-L-lysine) Microscopy Chamber* (Lot: 120502/3; Ibidi, Martinsried, Germany) in each chamber in a volume of 500µL each. Incubation was over night at 37°C/5%CO₂ in a humidified atmosphere. 4 of 8 chambers were infected with RV-TB40/E-delUL16EGFP at an m.o.i. of 1 in a volume of 100µL. 4 of 8 chambers were carried along as mock controls. Fixation was performed at 3 days post infection. For this, culture supernatant was discarded and the cells were washed once in cold 1XPBS. Following that paraformaldehyde [(P6148-500g; #109K1434; Sigma-Aldrich) + 100mL 1XPBS] was added and left on the cells for 20 minutes. After that, paraformaldehyde was removed and the cells were washed 3 times in 1XPBS. Cells were then resuspended in 1XPBS and stored at 4°C until analysis. Cells were then stained with the "In Situ Cell Death Detection Kit, Fluorescein (Roche, Cat. No.1 684 795) according to the manufacturer's instructions. Analysis was performed by inspection through an Axiophot microscope (Zeiss), using fluorescent light. No indication of apoptosis following infection could be detected; infected and mock-infected cells showed equal fluorescence signals.

### Example 12: Titration of virus stocks

1.8 x 10⁶ HFF were seeded in a 175cm² culture flask and were incubated over night at 37°C/5%CO₂ in a humidified atmosphere. The following day, the cells were infected with 1mL virus stock. Virus supernatant was collected at day 7 after infection and used to infect 10-20 175cm² culture flasks with 1.8 x 10⁶ HFF. 7 days after that, culture supernatant was again collected and apportioned in volumes of 1-1.5mL. Virus stocks were frozen at -80°C until further use.

For titration, 5 x 10³ in 50µL HFF were seeded in each well of a 96 well-plate and incubated overnight. 10 fold serial dilutions of the respective virus stocks were applied to the different wells. Each sample was tested in quadruplicate. Incubation was for 2 days at 37°C/5%CO₂ in a humidified atmosphere. After that, supernatant was discarded and cells were washed with 1xPBS. Fixation was with 100µL 96% vol/vol ethanol. After that, ethanol was discarded and cells were washed again with 1xPBS.

Residual PBS was removed by tapping the plate on a towel. Subsequently, 50µL undiluted supernatant of the antibody p63-27 (IE1-pp72) was applied to each well. Incubation was for 1 hour at 37°C in a wet chamber. After that the primary antibody was removed by tapping the plate on a towel and the plate was rinsed again with 1xPBS. After that, an anti-mouse antibody, coupled to horse-radish peroxidase, was applied. Incubation was again for 1 hour at 37°C in a wet chamber. After the removal of the secondary antibody solution and two PBS-washing steps, AEC substrate was applied. AEC substrate was diluted 1:20 in acetate-buffer and was subsequently filtrated through a MN 615 Ø0185mm filter paper (Macherey-Nagel). H₂O₂ (hydrogen peroxide 30% Art.-Nr. 8070.1; Roth) was added to the substrate at a dilution of 1:1,000 immediately before application. Incubation was for 1 hour at 37°C in a wet chamber. After removal of the substrate, cells were again rinsed twice with PBS and were stored at 4°C until inspection. The number of positive nuclei was then taken as a measure for infectivity (m.o.i.).

### Solutions:

Acetate-buffer: 13.6 g Na-Acetat x 3 H₂O
   2.88mL glacial acetic acid
   ad 1000mL H₂O
   pH = 4.9

AEC-Stock:
- Apply 10 AEC-tablets (≡ 200mg) in a 50ml Falcon tube.
   (3-Amino-9-Ethyl-Carbazole (AEC) Tablets (No. 205-057-7, Sigma, Cat.-No. A6926)
- dissolve in 50 ml DMF (n,n-Dimethylformamide; Sigma, Cat.-No. D4254, 250 ml) by vortexing
- dispense in 1mL Aliquots in 1.5mL-tubes; store at: -20°C

## Claims

1. Method for the production of human Cytomegalovirus (HCMV) particles, the method including the steps of:
(a) contacting and thereby infecting a permanent human amniocyte cell with HCMV,
(b) incubating the amniocyte cell,
(c) allowing expression of HCMV particles, and
(d) isolating the HCMV particles,
wherein the permanent human amniocyte cell expresses the adenoviral gene products E1A and E1B.

2. The method according to claim 1, wherein the amniocyte cells in step (a) are cultured in serum free medium.

3. The method according to claim 1 or 2, wherein the amniocyte cells in step (a) are cultured in serum-containing medium.

4. The method according to any one of the previous claims 1 to 3, wherein the amniocyte cells are adherent cells or suspension cells.

5. The method according to any one of the previous claims 1 to 4, wherein the HCMV particles in step (c) are isolated from the medium or from the intracellular space of the amniocyte cell.

6. The method according to any one of the previous claims 1 to 5, wherein the isolating in step (d) is conducted from the medium with rate velocity gradient centrifugation, density gradient differential centrifugation or zone centrifugation.

7. The method according to any one of the previous claims 1 to 6, wherein the permanent human amniocyte cell is grown in the lag phase, the exponential phase or the stationary phase during the time of the contacting and infecting with HCMV in step (a).

8. The method according to any one of the previous claims 1 to 7, wherein the adenoviral gene products E1A und E1B comprise the nucleotide 1 to 4344, 505 to 3522 or nucleotide 505 to 4079 of the human adenovirus serotype-5.

9. The method according to any one of the previous claims 1 to 8, wherein the permanent human amniocyte cell expresses the adenoviral the adenoviral gene product pIX.

10. The method according to any one of the previous claims 1 to 9, wherein the amniocyte cell is contacted and infected in step (a) with HCMV in an amount in the range of 0.001 to 10 MOI.

11. HCMV particles produced according to a method according to any one of the claims 1 to 10.

12. A HCMV based vaccine comprising HCMV particles according to claim 11.

13. Use of HCMV particles according to claim 11 for the preparation of a HCMV based vaccine.

14. Use of HCMV particles according to claim 13, wherein the HCMV particles are placed into a pharmaceutically acceptable composition.

15. HCMV particles according to claim 11 for use in the preparation of a therapeutic or diagnostic agent for the prevention or treatment of HCMV related disease.
